# EUROPÄISCHE PATENTSCHRIFT

(11) **EP 1 774 082 B1**
(45) Veröffentlichungstag und Bekanntmachung des Hinweises auf die Patenterteilung: **17.06.2009**
(21) Anmeldenummer: 05772613.5
(22) Anmeldetag: 15.07.2005
(51) Int. Cl.: D06H 3/16, G01N 3/10, G01N 3/08, G01L 1/04, G01M 19/00

(54) **VORRICHTUNG ZUM BESTIMMEN VON PARAMETERN VON INSBESONDERE THERAPEUTISCHEN KOMPRESSIONSMASSNAHMEN AN GLIEDMASSEN**
DEVICE FOR THE DETERMINATION OF PARAMETERS PARTICULARLY FOR THERAPEUTIC COMPRESSION MEASURES ON LIMBS
DISPOSITIF POUR DETERMINER DES PARAMETRES EN PARTICULIER POUR DES MESURES DE COMPRESSION THERAPEUTIQUE SUR DES MEMBRES

(30) Priorität: 30.07.2004 DE 102004038421
(43) Veröffentlichungstag der Anmeldung: 18.04.2007
(73) Patentinhaber: Paul Hartmann Aktiengesellschaft, 89522 Heidenheim (DE)
(72) Erfinder: WESP, Hansjörg, 89551 Königsbronn (DE); OESTREICHER, Ulrich, 37269 Eschwegen (DE); JUNG, Harald, 67757 Kreimbach-Kaulbach (DE)
(74) Vertreter: Langöhrig, Angelika Beate
(86) Internationale Anmeldenummer: PCT/EP2005/007695
(87) Internationale Veröffentlichungsnummer: WO 2006/012986

(56) Entgegenhaltungen:
- FR-A- 2 854 694
- GB-A- 2 168 156
- US-A- 2 675 703
- US-A- 3 818 756
- US-B1- 6 334 363

## Beschreibung

Die Erfindung betrifft eine Vorrichtung zum Bestimmen von Parametern von insbesondere therapeutischen Kompressionsmaßnahmen an Gliedmaßen umfassend eine anatomisch nachgebildete Gliedmaße, an der die Kompressionsmaßnahme anbringbar ist, wobei an der Gliedmaße Sensoren vorgesehen sind zur Aufnahme der zu bestimmenden Parameter.

Derartige Kompressionsmaßnahmen können beispielsweise sogenannte "Kompressionsstrümpfe" oder "Kompressionsstrumpfhosen", allgemein Strümpfe oder Strumpfhosen mit einer gewissen Stützwirkung, aber auch entsprechende, zumeist schlauchförmige Gebilde für den Armbereich sein, wo derartige Maßnahmen beispielsweise nach Brustoperationen eingesetzt werden, um Flüssigkeitsansammlungen in den Armen zu verhindern bzw. zu beheben und schließlich können derartige Kompressionsmaßnahmen auch durch Flachmaterialien, wie beispielsweise Bandagen oder Binden erzielt werden, die zum Aufbringen einer Kompression an der gewünschten Gliedmaße angebracht werden. Derartige Produkte sind zumeist Gestricke, wobei für den Erfolg der Therapie es von entscheidender Bedeutung ist, dass ausreichende, aber nicht zu hohe Drücke an der Körperoberfläche erzielt werden. Die Höhe des Kompressionsdruckes hängt insbesondere von den Eigenschaften des Materials, der Verarbeitung bzw. Fertigungstechnik und der Anlagetechnik ab. Die Aufnahme von Parametern, insbesondere der aufgebrachten Kompression bzw. des Drucks, ist zum einen für die Herstellung bzw. Entwicklung entsprechender Kompressionsmaßnahmen von Vorteil, kann zum anderen jedoch auch beispielsweise zum Training von Patienten, bei denen entsprechende Kompressionsmaßnahmen eingesetzt werden sollen bzw. zum Training von medizinischem und therapeutischem Personal verwandt werden.

Über Kraftdehnungsbeziehungen eines spezifischen Kompressionsmaterials und Anwendung der Formel nach Laplace kann theoretisch berechnet werden, welche Punktdrücke in Abhängigkeit des Radius des Untergrunds resultieren. Dies gilt für zylindrische und starre Körper.

Derartige starre Systeme sind im Stand der Technik bereits vielfach beschrieben worden. So ist beispielsweise aus der US-PS 6,334,363 B1 eine Vorrichtung zur Messung von Druckpunkten durch therapeutische Kompressionsmaßnahmen bekannt, wobei eine Vielzahl von Sensoren vorgesehen ist, die auf der Oberfläche der starren, einem Bein entsprechenden Form angebracht sind. Durch die gleichzeitige Messung der Drücke an allen Messpunkten und die Vielzahl der Messpunkte kann ein Druckprofil der Kompressionsmaßnahme erzeugt werden.

Des Weiteren offenbart die US-PS 4,137,763 ein starres System, bei dem ebenfalls Druckwerte an einer Vielzahl von Messpunkten aufgenommen werden können.

Der menschliche Unterschenkel oder auch der menschliche Arm als die Hauptanwendungsgebiete für Kompressionsmaßnahmen sind jedoch weder streng zylindrisch noch starr. Vielmehr entstehen, je nach Ausprägung der Muskulatur und Beweglichkeit des oberen Sprunggelenks bzw. Knies, Verschiebungen der Muskelbäuche bei der Muskelkontraktion, z. B. beim Laufen. Damit entsteht ein dynamisches System, bei dem die Umfänge am Unterschenkel mit jedem Schritt annähernd zyklisch verändert werden.

Davon betroffen sind die resultierenden Drücke unter einer Kompressionsmaßnahme, die sich entsprechend mit dem Schrittzyklus ändern. Bei erschlafftem Muskel spricht man vom Ruhe-, bei kontrahiertem vom Arbeitsdruck. Das Verhältnis Ruhe- zu Arbeitsdruck einer Kompressionsmaßnahme ist die für die klinische Wirksamkeit der Therapie entscheidende und anerkannte Größe.

Einen ersten Ansatz zur Verbesserung derartiger Vorrichtungen zeigt beispielsweise die EP 1 118 851 A1, die eine Vorrichtung zum Messen der Kompression durch Strumpfwarenartikel offenbart, bei der ein Unterrumpf vorgesehen ist aus einzelnen röhrenförmigen Elementen, die zum Teil aus Schalen aufgebaut sind, wobei die Schalen sich aufspreizen lassen, um Stärkenvariationen entlang der Länge eines Beins nachbilden zu können. Auf diese Weise können erste elastische Eigenschaften einer Strumpfware aufgenommen werden. Nachteilig ist jedoch hierbei, dass die Nachbildung des Bewegungsapparates aufgrund der einzelnen röhrenförmigen Elemente sehr abstrahiert ist und darüber hinaus durch die mechanische Aufspreizung der röhrenförmigen Segmente eine Simulation eines Bewegungsablaufes nur sehr unvollkommen möglich ist.

Darüber hinaus ist eine Aufspreizung, jedoch zur Anpassung des Messkörpers an eine zu messende Strumpfware, aus der GB 2,168,156 A1 vorbekannt.

Weiterhin war es im Stand der Technik bekannt, an Menschen selbst Messungen zum dynamischen Druckverhalten durchzuführen, indem man Druckaufnehmer auf der Haut platziert hat, die Patienten mit einer entsprechenden Kompression versorgt wurden und dann auf einem Laufband gehen mussten, während die Drücke kontinuierlich aufgenommen wurden. Derartige Messungen sind zwar praxisnah, jedoch typischerweise nur schwer reproduzierbar oder übertragbar.

Aufgrund der Tatsache, dass bei einzelnen Personen eine erhebliche Varianz zwischen beiden Beinen sowie von Tag zu Tag bzw. auch über den Tag besteht, lässt sich auch bei wiederholten Messungen die Präzision in vivo nur stark begrenzt an den erwünschten Wert annähern.

Es ist daher Aufgabe der Erfindung, eine Vorrichtung der eingangs beschriebenen Art bereitzustellen, welche eine reproduzierbare, quasi kontinuierliche Messung von Drücken sowohl statisch als auch dynamisch von Kompressionsmaßnahmen erlaubt.

Die Erfindung löst dabei die Aufgabe durch eine Vorrichtung der eingangs beschriebenen Art, bei der die Oberfläche der Gliedmaße zumindest partiell in mindestens eine Richtung elastisch verformbar ist und in der Gliedmaße mindestens eine Simulationseinrichtung für einen Muskel vorgesehen ist, der zur lediglich partiellen Verformung der Oberfläche der Gliedmaße ansteuerbar ist.

Auf diese Weise kann ein möglichst Nahekommen an die tatsächliche Gestaltung einer menschlichen Gliedmaße vorgesehen sein. Sofern es sich um ein Bein als Gliedmaße handelt, kann die Gliedmaße in ihrer äußeren Form einem Standardbein (Standardmaße von Strumpfherstellern, aber auch anderen bekannten Maßen zur Herstellung eines möglichst der menschlichen Natur nahekommenden Beins) nachgebildet sein. Dabei kann bei einer Nachbildung insbesondere vorgesehen sein, dass ein oder zwei Simulationseinrichtungen für Muskeln insbesondere im Bereich der Wadenmuskulatur (Musculus triceps surae, Musculus gastrocnemius und/oder Musculus soleus) angeordnet sind. Sofern als Gliedmaße ein Arm nachgebildet werden soll, können z. B. Bizeps oder Trizeps nachgebildet werden. Dabei kann vorgesehen sein, das die Gliedmaße nicht vollständig, sondern beispielsweise lediglich ein Teil der Gliedmaße, wie Unterarm oder Unterschenkel, nachgebildet werden. Alternativ können auch vollständige Gliedmaßen dargestellt werden.

Um eine besonders gute Nachbildung einer Gliedmaße zu erreichen, können mindestens zwei Simulationseinrichtungen für Muskeln vorgesehen sein, die jeweils lediglich eine partielle Verformung der Oberfläche der Gliedmaße hervorrufen. Hierbei kann es interessant sein, dass die mindestens zwei Muskeln nicht gleichzeitig, sondern zumindest zum Teil unabhängig und insbesondere alternierend ansteuerbar sind, so dass durch sie eine Muskelkontraktion bzw. -relaxion nachgebildet wird bzw. Bewegungen, wie das Laufen, simuliert werden. Durch diese Nachbildung der Muskelrelaxion bzw. - kontraktion wird die partielle Verformung der Oberfläche der Gliedmaße hervorgerufen.

Dabei kann insbesondere vorgesehen sein, dass nicht alle Simulationseinrichtungen für Muskeln gleichzeitig, sondern zumindest zum Teil unabhängig und insbesondere alternierend ansteuerbar sind, wobei durch die Ansteuerung dann eine partielle Verformung der Oberfläche der Gliedmaße erfolgt.

Dabei können die Simulationseinrichtungen für die Muskeln einen Hohlkörper umfassen, der volumen- und/oder formveränderlich ist, wobei die Volumen- und/oder Formveränderung durch Entleeren bzw. Befüllen des Hohlkörpers erfolgt und durch die verschiedenen Füllstände die partielle Verformung der Oberfläche der Gliedmaße hervorgerufen wird. Dabei kann insbesondere vorgesehen sein, dass die Simulationseinrichtungen der Muskeln über Schläuche mit Luft bzw. Flüssigkeiten befüllt werden können. Die Elastizität der Oberflächenmaterialien erlaubt dabei eine Dickenausdehnung der Gliedmaße im Bereich über den simulierten Muskeln. Auf diese Weise können sämtliche Relaxions- bzw. Kontraktionszustände des nachgebildeten Muskels simuliert werden. Dabei kann vorgesehen sein, dass auch die Umhüllung des Hohlkörpers elastisch gestaltet ist. Sofern weitere Materialien zwischen dem simulierten Muskel und der Oberfläche der Gliedmaße vorgesehen sind, können diese ebenfalls elastifiziert sein.

Es kann weiterhin vorgesehen sein, dass der Luft- bzw. Flüssigkeitsstrom hinsichtlich Gesamtvolumen und Volumenstrom eingestellt werden kann. Auf diese Weise kann die Geschwindigkeit und das Ausmaß der Volumenverschiebung bei der Muskelkontraktion eingestellt werden.

Mit einem derartigen System können neben den bereits genannten Vorteilen folgende weitere Vorzüge erzielt werden: So kann ein derartiges System neben der Qualitätskontrolle in der Fertigung entsprechender Kompressionsmittel auch der Qualitätskontrolle nach langer Belastung oder mit gealterten oder sonstigen veränderten Materialien dienen. Darüber hinaus kann ein entsprechendes System zur Entwicklung neuer Kompressionsmaterialien bzw. -kombinationen und im direkten Vergleich von Produkten eingesetzt werden. Weitere Anwendungsmöglichkeiten sind die physiologische Charakterisierung von Kompressionsmaterialien, aber auch die Qualitätssicherung in der Pflege und Anwendung, da beispielsweise Schwestern, aber auch Patienten den Erfolg der Anlegetechnik direkt kontrollieren können. Auf diese Weise kann die therapeutische Maßnahme in ihrer Wirksamkeit verbessert werden.

Es kann dabei vorgesehen sein, dass die Oberfläche der Gliedmaße mit einer synthetischen Haut bezogen ist. Eine derartige synthetische Haut wird in der Regel für prothetische Maßnahmen als Oberfläche verwendet. Darüber hinaus kann vorgesehen sein, dass Simulationseinrichtungen für Knochen und/oder Weichteile und/oder Gelenke vorgesehen sind. So kann beispielsweise vorgesehen sein, im Fußbereich mittels Holz oder Metallelementen die Ferse nachzubilden.

Ebenfalls kann eine Simulationseinrichtung für das Schienbein vorgesehen sein.

Als Simulationseinrichtungen für die Weichteile können Elemente aus Silikon vorgesehen sein, wobei Silikone mit verschiedenen Elastizitätskoeffizienten verwendet werden. Dabei kann vorgesehen sein, dass, abgesehen von technisch notwendigen Bestandteilen, wie Zuführungsleitungen, Knochen und Muskelelementen, die übrige Form vollständig aus Silikon bzw. Mischungen oder verschiedenen Silikonen hergestellt ist. Dabei kann die Herstellung derart erfolgen, dass zunächst anhand von Schnittbildern, beispielsweise eines Standartbeines (Standardmaße von Stumpfherstellern), eine Umrechnung der hierdurch erlangten Schnittbilder auf das 50. Perzentil Mann erfolgt. Die einzelnen Schnittbilder werden dann zur Erstellung eines Modells auf Hartschaumscheiben aufgeklebt und ausgeschnitten und die Scheiben aufeinander fixiert und die Übergänge geglättet. Von dem Hartschaummodell wird dann eine Negativform aus Gips erstellt und Silikon in die Negativform mit Aussparungen für die Bauteile gegossen. Es erfolgt dann der Einbau der Bauteile, wie Hohlkörper für die Muskeln, Gestänge sowie weitere Simulationseinrichtungen für Knochen, etc. Anschließend wird die Außenstruktur, nämlich die synthetische Haut, aufgebracht. Bei dem verwendeten Silikon kann es sich um das Silikon der Marke Elastosil der Firma Wacker-Chemie GmbH handeln, das aufgrund seiner Verarbeitungseigenschaften, Viskosität und Materialeigenschaften im ausgehärteten Zustand ausgewählt werden kann. Dabei kann insbesondere auch ein Schichtaufbau aus verschiedenen zentrisch aufgebauten Silikonschichten vorgesehen sein.

In einem bevorzugten Beispiel der Erfindung kann vorgesehen sein, dass der Schichtaufbau aus Silikonen unterschiedlicher Härte hergestellt wird. So kann beispielsweise im Inneren der Vorrichtung ein Silikon der Härte Shore A von größer 15 (DIN 53505) verwendet werden, wogegen die auf die innere Schicht zu liegende Schicht ein Silikon der Härte Shore A von kleiner 15 (DIN 53505) umfasst. Insbesondere ist vorgesehen, dass die Simulationseinrichtung für den oder die Muskel von einem Silikon hoher Elatizität und mechanischer Festigkeit umgeben ist.

Die vorgesehenen Sensoren können unter der synthetischen Haut oder in das Hautersatzmaterial eingelassen angeordnet sein. So kann vorgesehen sein, dass an den jeweiligen Stellen Ausstanzungen im Hautersatzmaterial vorgesehen sind, in die die Sensoren, insbesondere Piezo-Kraftaufnehmer, eingelassen sind. Die Kabel und Zuleitungen bzw. Ableitungen für den Datentransport können unter der Kunsthaut verlegt werden und dann mit einer Auswerteeinrichtung derart verbunden sein, dass die ermittelten Daten einer Weiterverarbeitung und/oder Visualisierung und/oder Speicherung zur Verfügung gestellt werden können. Die Sensoren werden dabei in geeigneter Anzahl und Menge an der Gliedmaße angebracht.

Des Weiteren kann vorgesehen sein, dass die Gliedmaße ein oder mehrere Bereiche, insbesondere Gelenkbereiche, umfasst, die der Simulation von beweglichen Gelenken dienen. Dies ist insbesondere von Vorteil bei Strumpfwaren, die insbesondere auf die Kompressionseigenschaften im Übergang zwischen Bein und Fuß ausgelegt werden müssen, bzw. beim Übergang zwischen Unterschenkel zu Oberschenkel oder im Hüftbereich oder den Armgelenken.

Schließlich kann auch vorgesehen sein, dass Mittel in der Gliedmaße vorgesehen sind, die eine Vergrößerung des Gesamtumfangs der Gliedmaße ermöglichen, so dass die Gliedmaße an Schwankungen der menschlichen Struktur, wie sie beispielsweise über den Tag, aber auch von Tag zu Tag bzw. zwischen den Jahreszeiten und Temperaturen auftreten, angepasst werden kann.

Weitere Vorteile und Merkmale der Erfindung ergeben sich aus den übrigen Anmeldungsunterlagen. Die Erfindung soll im Folgenden anhand einer Zeichnung näher erläutert werden. Dabei zeigen:
- Figur 1: eine schematische Darstellung im Schnitt einer Gliedmaße der erfindungsgemäßen Vorrichtung in der Ausgestaltung eines Unterschenkels,
- Figur 2: einen Schnitt durch Figur 1 entlang der Line II-II,
- Figur 3: einen Schnitt durch Figur 1 entlang der Line III-III,
- Figur 4: einen Schnitt durch Figur 1 entlang der Linie IV-IV,
- Figur 5: eine vergrößerte schematische Darstellung der Oberfläche der Gliedmaße und
- Figur 6: eine Vorrichtung gemäß Figur 1 mit der zugehörigen Steuerungs- und Auswerteeinheit.

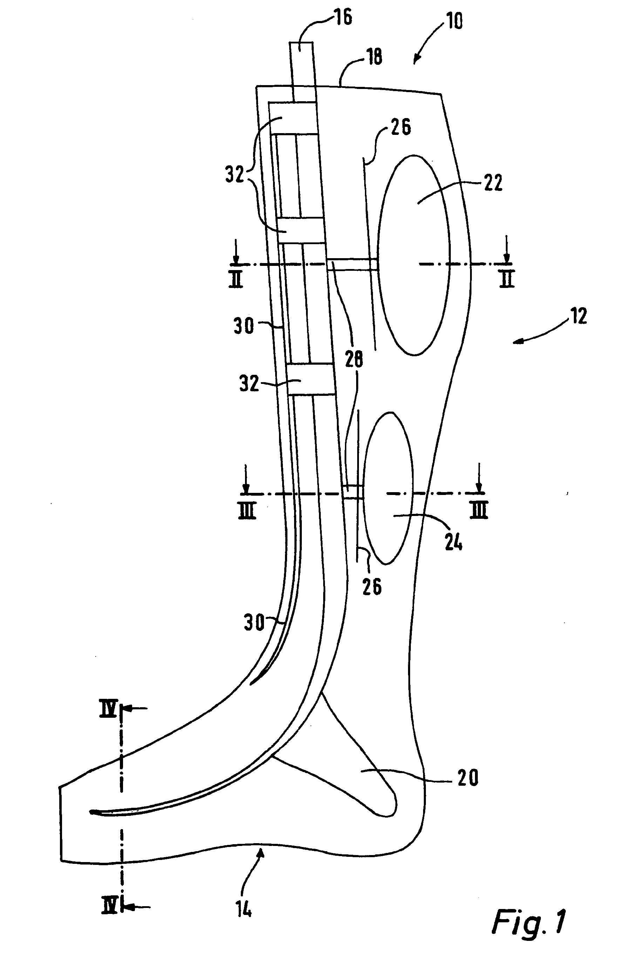

Figur 1 zeigt eine Gliedmaße, nämlich einen Unterschenkel, der eine anatomisch nachgebildete Form aufweist, wobei die zur Verfügung stehenden Daten für den Nachbau der Gliedmaße am Bein einer Person ermittelt, standardisiert und dann auf das vorliegende Modell übertragen wurden. Die Gliedmaße 10 umfasst dabei einen Wadenbereich 12 sowie einen Fußbereich 14. Die Verbindung zwischen Wadenbereich 12 und Fußbereich 14 ist beim vorliegenden Modell unbeweglich, d. h., es sind keine Simulationseinrichtungen für ein Gelenk vorgesehen. Durch die gesamte Gliedmaße 10 zieht sich dabei ein Versorgungsrohr 16, das an der Oberkante 18 der Gliedmaße 10 in die Gliedmaße 10 eintritt und bis in den Fußbereich 14 verläuft. Das Versorgungsrohr 16 dient neben der noch später erläuterten Versorgung sowohl der Simulationseinrichtungen für die Muskeln 22, 24 oder Muskelnachbildungen 22, 24 als auch der Sensoren der Stabilisierung der Gliedmaße 10. Dabei verjüngt sich das Versorgungsrohr 16 im Bereich des Fußes 14, in dem es bei der vorliegenden Gliedmaßengestaltung lediglich Haltefunktion ausübt, da bei der vorliegenden Gestaltung im Bereich des Fußes 14 keine Ermittlung der Kompressionsmaßnahmen bezüglich des Arbeitsdruckes erfolgen soll. Das Versorgungsrohr 16 weist dabei im Bereich des Fußes 14 einen rechteckigen Querschnitt auf, wobei im Bereich des Unterschenkels 12 das Versorgungsrohr 16 einen runden Querschnitt besitzt.

Das Versorgungsrohr 16 ist im Bereich der Vorderseite des Wadenbereichs, also in der Nähe eines bei einem Menschen vorgesehen Schienbeins, angeordnet.

Das Versorgungsrohr 16 dient weiterhin zur Halterung weiterer Bauteile der Vorrichtung. So ist beispielsweise in einem Fersenbereich ein hölzernes Element als Ersatz eines Fersenbeins 20 angebracht, um die Fußform der Vorrichtung zu stabilisieren.

Die Gliedmaße umfasst des Weiteren zwei Hohlkörper, deren Wandung aus einem luftundurchlässigen elastischen Material gebildet ist. Die Hohlkörper werden durch zwei Druckluftkissen der Fa. Pronal - Leers (FR) dargestellt. Die Hohlkörper dienen als Simulationseinrichtungen für Muskeln, hier für die beiden wesentlichen Wadenmuskeln. Das Volumen der Hohlkörper kann dabei unterschiedlich sein.

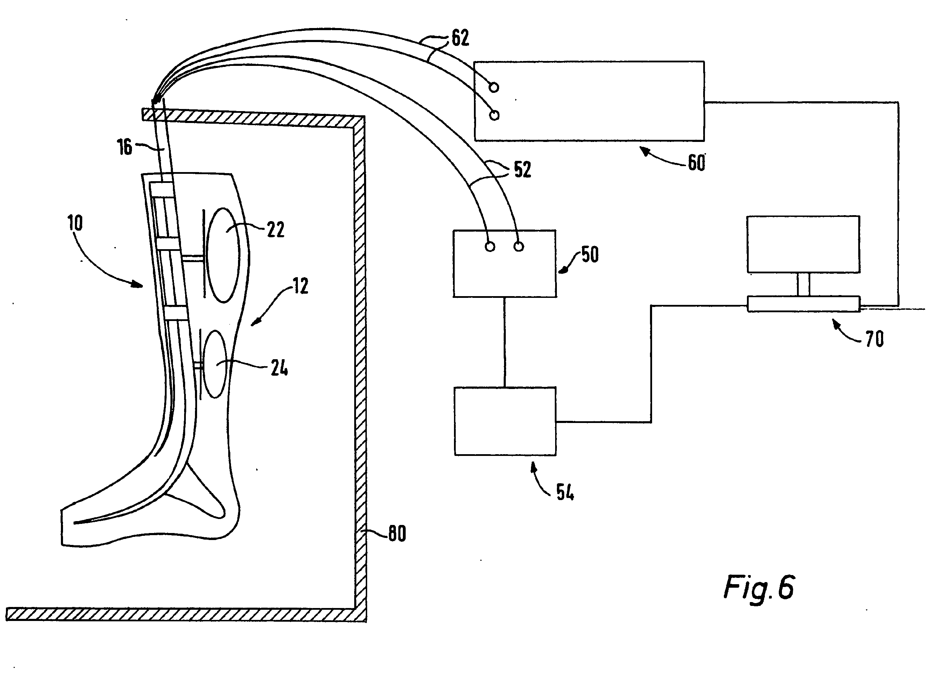

Die beiden durch die Nachbildungen simulierten Muskeln 22 und 24 sind dabei die Muskeln, die beim Gehen eines Menschen für die Verformung des Wadenbereichs 12 (Musculus gastrocnemius und Musculus soleus) eines Unterschenkels verantwortlich sind. Die Hohlkörper sind mittels Halteplatten 26 sowie Versorgungsrohre 28 mit dem Versorgungsrohr 16 verbunden. Im Versorgungsrohr 16 sowie den Versorgungsrohren 28 sind Luftzu- und -ableitungen geführt, die ein Befüllen und Entleeren der Hohlkörper ermöglichen. Hierbei wird das Befüllen zumeist alternierend zwischen den beiden Hohlräumen durchgeführt werden, da dies den Gehvorgang am besten annähert. Die Zu- und Ableitungen für die Luftbefüllung können dabei mit einem externen Kompressor 50 (Figur 6) verbunden sein. Darüber hinaus ist mit dem Versorgungsrohr 16 eine Simulationseinrichtung für einen Schienbeinknochen 30 verbunden, der sich entsprechend eines menschlichen Schienbeins im vorderen Kantenbereich über die Länge der Extremität erstreckt. Zur Halterung der Schienbeinnachbildung 30 sind Halteeinrichtungen 32 vorgesehen, die jeweils die Schienbeinnachbildung 30 mit dem Versorgungsrohr 16 verbinden.

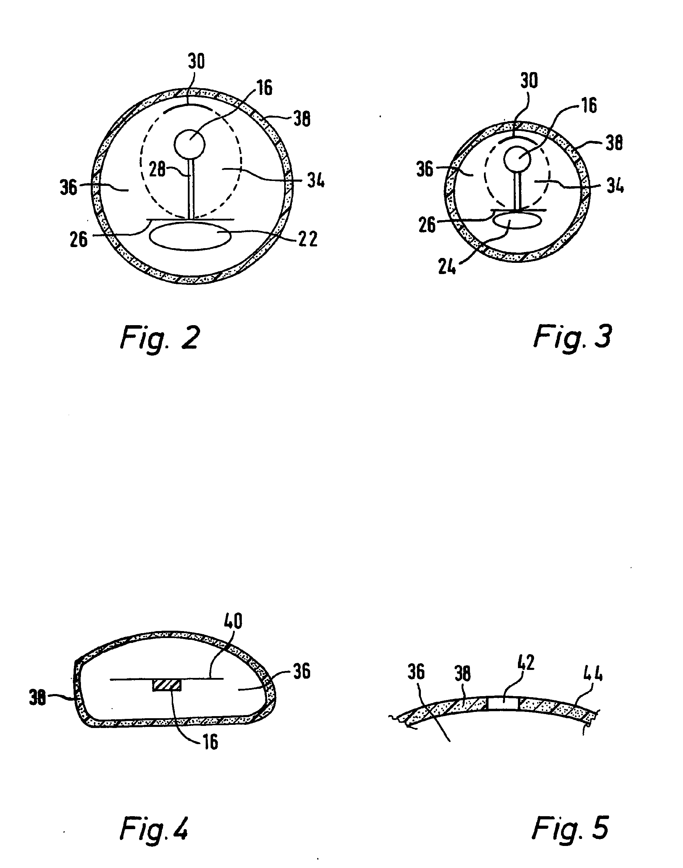

Der weitere Aufbau der Gliedmaße soll nun anhand der Figuren 2 bis 5 näher erläutert werden. Figur 2 zeigt einen Schnitt entlang der Linie II-II, wobei sich das Versorgungsrohr 16 im vorderen Bereich der Gliedmaße, außerzentrisch befindet. In einigem Abstand zum Versorgungsrohr 16 ist in Richtung auf die Vorderkante, d. h. das Schienbein der künstlichen Gliedmaße 10, die Schienbeinnachbildung 30 angeordnet. In Richtung der Wade erstreckt sich dagegen, ausgehend vom Versorgungsrohr 16, das Versorgungsrohr 28 bis zum Hohlkörper der Muskelnachbildung 22, wobei dieses Hohlkörper der an der Halteplatte 26 festgelegt ist und dort mit dem Versorgungsrohr 28 gekoppelt wird. Die Halteplatte 26 kann dabei schaumstoffummantelt ausgebildet sein. Zwischen der Schienbeinnachbildung 30 sowie der Halteplatte 26 befindet sich dabei das Versorgungsrohr 16 sowie das Versorgungsrohr 28 einschließend eine erste Weichteilsimulationseinrichtung 34, die aus einem ersten Silikon (Elastosil M 3500 Fa. Wacker Chemie GmbH (DE)) gebildet ist und sich zumindest im Wadenbereich 12 über die Länge der Gliedmaße 10 erstreckt. Die erste Weichteilsimulationseinrichtung 34 kann dabei aus einem ersten Silikonmaterial hergestellt sein, das einen ersten Elastizitätskoeffizienten aufweist. Darüber hinaus dient dieses erste Silikonmaterial dazu, das Versorgungsrohr 16 sowie das Versorgungsrohr 28 zu schützen und zu stabilisieren. Weiterhin wird durch die erste Silikonanordnung 34 die Schienbeinnachbildung 30 getragen.

Das verbleibende Volumen der Vorrichtung ist dann mit einem weiteren Silikonmaterial 36 (Elastosil M 4511 Fa. Wacker Chemie GmbH (DE)) ausgefüllt, das ebenfalls als Simulationseinrichtung für Weichteile dient. Dabei kann das zweite Silikonmaterial 36 weniger stabil, sondern elastischer und verformbarer als das erste Silikonmaterial 34 sein. Die äußere Oberfläche der Vorrichtung wird dann durch eine synthetische Haut 38 (Softtouch - Überziehstrumpf, Otto Bock - Duderstade (DE)) gebildet, die die Vorrichtung vollständig umschließt. Figur 3 ist dabei entsprechend aufgebaut.

Im Bereich des Fußes 14, wobei es sich anhand der zu erkennenden verschiedenen Spannhöhe um ein Modell für einen linken Fuß handeln kann, ist am Versorgungsrohr 16, das hier im Wesentlichen aus einem Vollmaterial besteht, lediglich noch eine Halteplatte 40 für den Fuß angebracht. In dem Fußbereich ist ein Silikonmaterial 37 (Elastosil M 4511 Fa. Wacker Chemie GmbH (DE)) vorgesehen, das eine noch größere Stabilität aufweist, als das erste Silikonmaterial 34.

Figur 5 zeigt nun eine Gestaltung der Haut 38, bei der es sich um eine Kunsthaut handelt, wie sie üblicherweise für die Gliedmaßenprothetik eingesetzt wird. Dabei wird zum Einsetzen von Sensoren 42 (Drucksensoren Fa. Gisma GmbH - Buggingen (DE)) jeweils ein Stück aus der Kunsthaut 38 ausgestanzt und hier ein Sensor, insbesondere ein Piezo-Kraftaufnehmer, eingesetzt, und zwar derart, dass die Oberfläche des Sensors 42 mit der Oberfläche 44 der Kunsthaut 38 abschließt. Die elektrische Versorgung sowie die Datenleitungen werden unterhalb der Kunsthaut 38 insbesondere zwischen der Kunsthaut 38 und dem Silikon 36 entlanggeführt und dann an geeigneter Stelle in das Versorgungsrohr 16 eingeführt.

Figur 6 zeigt nun die vollständige Vorrichtungsanordnung zur Messung der Kompression beispielsweise eines bis zum Knie reichenden Kompressionsstrumpfes. Dabei wird der Kompressionsstrumpf (nicht dargestellt) über die Gliedmaße 10 gezogen, wie eine später den Kompressionstrumpf tragenden Person diesen anlegen würde. Die zuvor beschriebenen Sensoren 42 sind hierbei zumindest im gesamten Wadenbereich 12 der Gliedmaße 10 über die Kunsthaut verteilt angeordnet. Sowohl die elektrische Versorgung als auch die Leitung für die aufzunehmenden Daten verläuft dabei zunächst zu einem A/D-Wandler 60 und von dort weiter zu einer computergestützten Auswerteeinheit 70.

Des Weiteren ist, wie bereits beschrieben, ein Kompressor 50 vorgesehen, der über Leitungen 52 mit den Muskelnachbildungen 22 und 24 in Verbindung steht. Die Ansteuerung der Muskelnachbildungen 22 und 24 erfolgt über eine Kompressorsteuerung 54, die für ein alternierendes Befüllen und Entleeren der Muskelnachbildungen 22 und 24 verantwortlich ist. Durch die Menge und den zeitlichen Ablauf der Befüllung und Entleerung kann ein Gehvorgang simuliert werden und es kann so neben dem aufzunehmenden statischen Druck im Ruhezustand des Beins auch die dynamische Druckverteilung der therapeutischen Kompressionsmaßnahme, hier des Kompressionsstrumpfes, gemessen werden. Die Messung erfolgt dabei über die ermittelten Werte der Sensoren 42, die diese über Datenleitungen 62 über den A/D-Wandler an die Auswerteeinheit 70 weitergeben. Ebenfalls mit der Auswerteeinheit 70 ist darüber hinaus die Kompressorsteuerung 54 verbunden.

Die Gliedmaße 10 kann über ihr Versorgungsrohr 16 an einem Rahmen 80 aufgehängt sein, um so eine Beeinflussung der Bewegung des Wadenbereichs 12 durch die Einflüsse einer Unterlage zu verhindern.

Die ermittelten Werte für die Kompressionsmaßnahme können dann in der Auswerteeinheit 70 visualisiert, gespeichert und weiterverarbeitet werden.

## Patentansprüche

1. Vorrichtung zum Bestimmen von Parametern von insbesondere therapeutischen Kompressionsmaßnahmen an Gliedmaßen (10) umfassend eine anatomisch nachgebildete Gliedmaße (10), an der die Kompressionsmaßnahme anbringbar ist, wobei an der Gliedmaße (10) Sensoren (42) vorgesehen sind zur Aufnahme der zu bestimmenden Parameter, **dadurch gekennzeichnet, dass** die Oberfläche (44) der Gliedmaße (10) zumindest partiell in mindestens eine Richtung elastisch verformbar ist und in der Gliedmaße (10) mindestens eine Simulationseinrichtung für einen Muskel (22, 24) vorgesehen ist, die zur lediglich partiellen Verformung der Oberfläche (44) der Gliedmaße (10) ansteuerbar ist.

2. Vorrichtung nach Anspruch 1, **dadurch gekennzeichnet, dass** mindestens zwei Simulationseinrichtungen für Muskel (22, 24) vorgesehen sind, die jeweils lediglich eine partielle Verformung der Oberfläche (44) der Gliedmaße (10) hervorrufen.

3. Vorrichtung nach Anspruch 2, **dadurch gekennzeichnet, dass** die mindestens zwei Simulationseinrichtungen für Muskeln (22, 24) nicht alle gleichzeitig sondern zumindest zum Teil unabhängig und insbesondere alternierend ansteuerbar sind.

4. Vorrichtung nach einem der vorangehenden Ansprüche, **dadurch gekennzeichnet, dass** die Simulationseinrichtung für den Muskel einen volumen- und/oder formveränderlichen Hohlkörper umfasst, der zur Volumen-und/oder Formveränderung entleerbar und/oder befüllbar ist und wobei durch die verschiedenen Füllstände die partielle Verformung der Oberfläche der Gliedmaße erzielbar ist.

5. Vorrichtung nach einem der vorangehenden Ansprüche, **dadurch gekennzeichnet, dass** die Simulationseinrichtungen für die Muskeln (22, 24) hydraulisch oder pneumatisch ansteuerbar sind.

6. Vorrichtung nach einem der vorangehenden Ansprüche, **dadurch gekennzeichnet, dass** die Oberfläche (44) der Gliedmaße durch eine synthetische Haut (38) gebildet ist.

7. Vorrichtung nach einem der vorangehenden Ansprüche, **dadurch gekennzeichnet, dass** die Gliedmaße (10) Simulationseinrichtungen für Knochen (20, 30) und/oder Weichteile (34, 36) und/oder Gelenke umfasst.

8. Vorrichtung nach Anspruch 6, **dadurch gekennzeichnet, dass** die Simulationseinrichtung für die Weichteile (34, 36) aus einem oder mehreren Silikonen hergestellt ist.

9. Vorrichtung nach einem der vorangehenden Ansprüche, **dadurch gekennzeichnet, dass** die Sensoren (42) unter der synthetischen Haut (38) und/oder in diese eingelassen angeordnet sind.

10. Vorrichtung nach einem der vorangehenden Ansprüche, **dadurch gekennzeichnet, dass** die Gliedmaße (10) ein oder mehrere Bereiche umfasst, die der Simulation der entsprechenden Gelenke dienen.

11. Vorrichtung nach einem der vorangehenden Ansprüche, **dadurch gekennzeichnet, dass** Mittel in der Gliedmaße (10) vorgesehen sind, die eine Vergrößerung des Gesamtumfangs der Gliedmaße (10) ermöglichen.

12. Vorrichtung nach einem der vorangehenden Ansprüche, **dadurch gekennzeichnet, dass** die Oberfläche (44) in Umfangsrichtung und/oder in alle Richtungen elastisch verformbar ist.

## Claims

1. Device for the determination of parameters of therapeutic compression measures on limbs (10) in particular, comprising an anatomically modelled limb (10) to which the compression measures may be applied, sensors (42) being provided on the limb (10) for recording the parameters to be determined, **characterised in that** the surface (44) of the limb (10) is at least partially resiliently deformable in at least one direction, and at least one simulation means for a muscle (22, 24) is provided in the limb (10) and may be activated to produce the merely partial deformation of the surface (44) of the limb (10).

2. Device according to claim 1, **characterised in that** at least two simulation means for muscles (22, 24) are provided and each produce merely a partial deformation of the surface (44) of the limb (10).

3. Device according to claim 2, **characterised in that** the at least two simulation means for muscles (22, 24) can be activated at least partially independently and in particular in alternation, rather than all simultaneously.

4. Device according to any one of the preceding claims, **characterised in that** the simulation means for the muscle comprises a cavity, which has a variable volume and/or shape and can be emptied and/or filled to provide the change in volume and/or shape, the different filling levels allowing the partial deformation of the surface of the limb to be achieved.

5. Device according to any one of the preceding claims, **characterised in that** the simulation means for the muscles (22, 24) can be activated hydraulically or pneumatically.

6. Device according to any one of the preceding claims, **characterised in that** the surface (44) of the limb is formed by synthetic skin (38).

7. Device according to any one of the preceding claims, **characterised in that** the limb (10) comprises simulation means for bones (20, 30) and/or soft tissue (34, 36) and/or joints.

8. Device according to claim 6, **characterised in that** the simulation means for the soft tissue (34, 36) is manufactured from one or more silicones.

9. Device according to any one of the preceding claims, **characterised in that** the sensors (42) are arranged under the synthetic skin (38) and/or are embedded therein.

10. Device according to any one of the preceding claims, **characterised in that** the limb (10) comprises one or more regions which simulate the corresponding joints.

11. Device according to any one of the preceding claims, **characterised in that** means are provided in the limb (10) to allow expansion of the overall size of the limb (10).

12. Device according to any one of the preceding claims, **characterised in that** the surface (44) is resiliently deformable in the peripheral direction and/or in all directions.

## Revendications

1. Dispositif destiné à déterminer des paramètres de mesures de compression en particulier thérapeutiques sur des membres (10), comportant un membre (10) reproduit de façon anatomique sur lequel la mesure de compression peut être placée, des capteurs (42) étant prévus sur le membre (10) afin d'enregistrer les paramètres à définir, **caractérisé en ce que** la surface (44) du membre (10) peut être au moins partiellement déformée élastiquement dans au moins une direction et **en ce qu'**il est prévu dans le membre (10) au moins un dispositif de simulation pour un muscle (22, 24) qui peut être commandé afin de déformer partiellement de façon vivante la surface (44) du membre (10).

2. Dispositif selon la revendication 1, **caractérisé en ce qu'**il est prévu au moins deux dispositifs de simulation pour muscles (22, 24) qui provoquent chacun de façon vivante une déformation partielle de la surface (44) du membre (10).

3. Dispositif selon la revendication 2, **caractérisé en ce que** les au moins deux dispositifs de simulation pour muscles (22, 24) peuvent être commandés non pas tous simultanément, mais au moins en partie indépendamment les uns des autres et en particulier en alternance.

4. Dispositif selon l'une des revendications précédentes, **caractérisé en ce que** le dispositif de simulation pour les muscles comporte un corps creux à volume et/ou forme variable qui peut être vidé et/ou rempli pour la modification du volume et/ou de la forme et la déformation partielle de la surface du membre pouvant être obtenue par les différents niveaux de remplissage.

5. Dispositif selon l'une des revendications précédentes, **caractérisé en ce que** les dispositifs de simulation pour les muscles (22, 24) peuvent être à commande hydraulique ou pneumatique.

6. Dispositif selon l'une des revendications précédentes, **caractérisé en ce que** la surface (44) du membre est formée par une peau synthétique (38).

7. Dispositif selon l'une des revendications précédentes, **caractérisé en ce que** le membre (10) comporte des dispositifs de simulation pour les os (20, 30) et/ou les parties molles (34, 36) et/ou les articulations.

8. Dispositif selon la revendication 6, **caractérisé en ce que** le dispositif de simulation pour les parties molles (34, 36) est fabriqué à partir d'une ou plusieurs silicones.

9. Dispositif selon l'une des revendications précédentes, **caractérisé en ce que** les capteurs (42) sont agencés sous la peau synthétique (38) et/ou incrustés dans celle-ci.

10. Dispositif selon l'une des revendications précédentes, **caractérisé en ce que** le membre (10) comporte une ou plusieurs zones qui servent à la simulation des articulations correspondantes.

11. Dispositif selon l'une des revendications précédentes, **caractérisé en ce qu'**il est prévu dans le membre (10) des moyens qui permettent une augmentation de la circonférence totale du membre (10).

12. Dispositif selon l'une des revendications précédentes, **caractérisé en ce que** la surface (44) peut être déformée élastiquement dans la direction circonférentielle et/ou dans toutes les directions.
